# EUROPEAN PATENT APPLICATION

(11) **EP 4 596 005 A1**
(43) Date of publication of application: **06.08.2025**
(21) Application number: 23869838.5
(22) Date of filing: 30.06.2023
(51) Int. Cl.: A61M 11/00

(54) **SPRAYER**

(30) Priority: 30.09.2022 CN 202211213795
(71) Applicant: Transpire Bio Inc, Weston, FL 33331 (US)
(72) Inventor: XIE, Pan, Shenzhen, Guangdong 518102 (CN); CHENG, Shiyi, Shenzhen, Guangdong 518102 (CN); LI, Jun, Shenzhen, Guangdong 518102 (CN)
(74) Representative: Latscha Schöllhorn Partner AG
(86) International application number: PCT/CN2023/104322
(87) International publication number: WO 2024/066590

(57) **Abstract**

An atomizer (10) comprises: a housing assembly (100), wherein the housing assembly (100) comprises a fixed shell (110); the fixed shell (110) comprises an indicator (114) and a plurality of guide teeth (1111) arranged in a circumferential direction, and a guide slot (1112) is formed between two adjacent guide teeth (1111); a jacking plate (300), slidably connected to the fixed shell (110) in the axial direction of the housing assembly (100); a counting plate (400), including a plurality of rotating teeth (410) arranged in the circumferential direction, wherein an receiving slot (414) is formed between two adjacent rotating teeth (410); the rotating teeth (410) match with the guide slots (1112); the guide teeth (1111) match with the receiving slots (414); the counting plate (400) is provided with scale markings (430) arranged in the circumferential directions; and a driving assembly (200), movably connected to the fixed shell (110) and resisting against a radial edge of the jacking plate (300), to periodically drive the jacking plate (300) to slide, causing the counting plate (400) to rotate and causing indicator (114) to point to different scale markings (430).

## Description

### TECHNICAL FIELD

This application is generally related to the technical field of inhalation devices, and in particular, to inhalation devices that atomize a liquid.

### BACKGROUND

Some types of inhalation devices, such as soft mist inhalers (hereinafter "atomizer"), spray an atomized liquid. To atomize the liquid, a liquid storage bottle containing a certain quantity of the liquid can be placed into the atomizer. The liquid in the liquid storage bottle can be medical liquid, or the like. Upon actuation, the atomizer sprays a quantity of the liquid. For traditional atomizers, when the liquid is sprayed from the atomizer and continuously consumed, a user cannot accurately know how much of the liquid remains in the liquid storage bottle, and thus cannot accurately know a volume of liquid that has been ingested. Consequently, there is a need to accurately determine a volume of remaining liquid in a liquid storage bottle in an atomizer.

### SUMMARY

In an aspect, an atomizer comprises a housing assembly, wherein the housing assembly includes a fixed shell; the fixed shell includes an indicator and a plurality of guide teeth arranged in a circumferential direction, and a guide slot is formed between two adjacent guide teeth; a jacking plate, slidably connected to the fixed shell in the axial direction of the housing assembly; a counting plate, including a plurality of rotating teeth arranged in the circumferential direction, wherein an receiving slot is formed between two adjacent rotating teeth; the rotating teeth match with the guide slots; the guide teeth match with the receiving slots; the counting plate is provided with scale markings arranged in the circumferential direction; and a driving assembly, movably connected to the fixed shell and resisting against a radial edge of the jacking plate, to periodically drive the jacking plate to slide, causing the counting plate to rotate and causing indicator to point to different scale markings.

Details of one or more embodiments of this application are provided in the accompanying drawings and descriptions below. Other features, objectives, and advantages of this application become apparent from the specification, the drawings, and the claims.

### BRIEF DESCRIPTION OF THE DRAWINGS

To better describe and explain the embodiments and/or examples disclosed herein, reference can be made to one or more accompanying drawings. The additional details or examples for describing the drawings should not be considered as limiting the scope of any of the disclosed applications, the embodiments and/or examples described so far, and the best modes of these applications currently understood.
FIG. 1 is a perspective view of a three-dimensional structure of an atomizer;
FIG. 2 is a cross-sectional view of a three-dimensional sectional structure of the atomizer shown in FIG. 1;
FIG. 3 is an exploded view of the atomizer shown in FIG. 1;
FIG. 4 is a cross-sectional and enlarged view of a three-dimensional structure of FIG. 3;
FIG. 5 is a cross-sectional and enlarged view of a first partially three-dimensional structure of the atomizer shown in FIG. 1;
FIG. 6 is a cross-sectional and enlarged view of a second partially three-dimensional structure of the atomizer shown in FIG. 1;
FIG. 7 is a cross-sectional and enlarged view of a third partially three-dimensional structure of the atomizer shown in FIG. 1;
FIG. 8 is perspective view of a three-dimensional structure of a rotating shaft in the atomizer in FIG. 1;
FIG. 9 is a perspective view of a three-dimensional structure of a jacking plate in the atomizer in FIG. 1;
FIG. 10 is a perspective view of a three-dimensional structure of a counting plate in the atomizer in FIG. 1; and
FIG. 11 is a perspective view of a three-dimensional structure of the counting plate shown in FIG. 10, viewed in another viewing angle.

### DETAILED DESCRIPTION

To facilitate understanding this disclosure, the disclosure will be described more comprehensively below with reference to related accompanying drawings. Different embodiments of this disclosure are provided in the drawings. However, the disclosure can be implemented in many different forms, and is not limited to the embodiments described herein. On the contrary, these embodiments are provided to make the content of this disclosure understood more thoroughly and comprehensively.

It should be noted that, when an element is referred to as being "fixed to" another component, the element can be integrally formed directly on that component or can be connected to that component via a connector or fastener. When an element is considered to be "connected to" another element, the element can be directly connected to the another component, or indirectly connected through an intermediate component. The terms "inner", "outer", "left", "right", and similar expressions used herein are for illustrative purposes only and do not necessarily represent the only implementation.

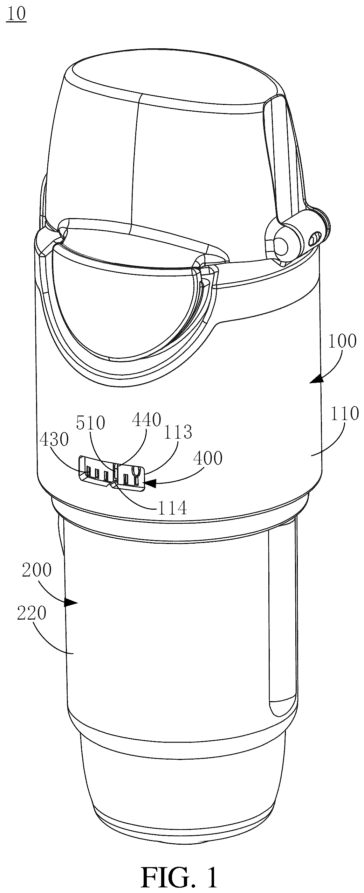

Referring to FIG. 1, FIG. 2, and FIG. 3, the atomizer 10 according to an embodiment can be used as a medical instrument, and a liquid storage bottle 20 containing liquid can be placed in the atomizer 10. In some cases, the liquid can contain a medicine. During operation, the atomizer 10 can atomize the liquid in the liquid storage bottle 20 to form a liquid mist or aerosol. A user can ingest the liquid by inhalation of the liquid mist. The liquid storage bottle 20 can further accommodate other liquids such as non-medicinal liquid. According to an embodiment, atomizer 10 can comprise a housing assembly 100, a driving assembly 200, a jacking plate 300, and a counting plate 400.

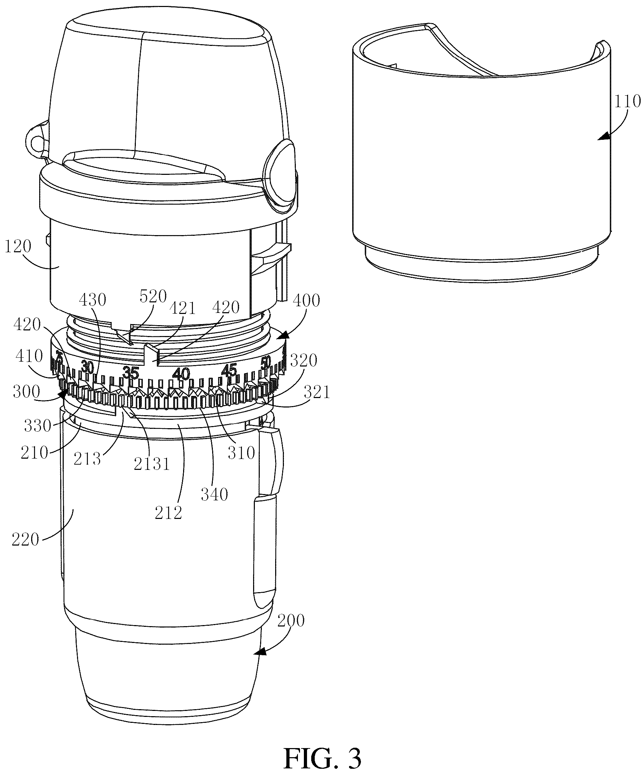

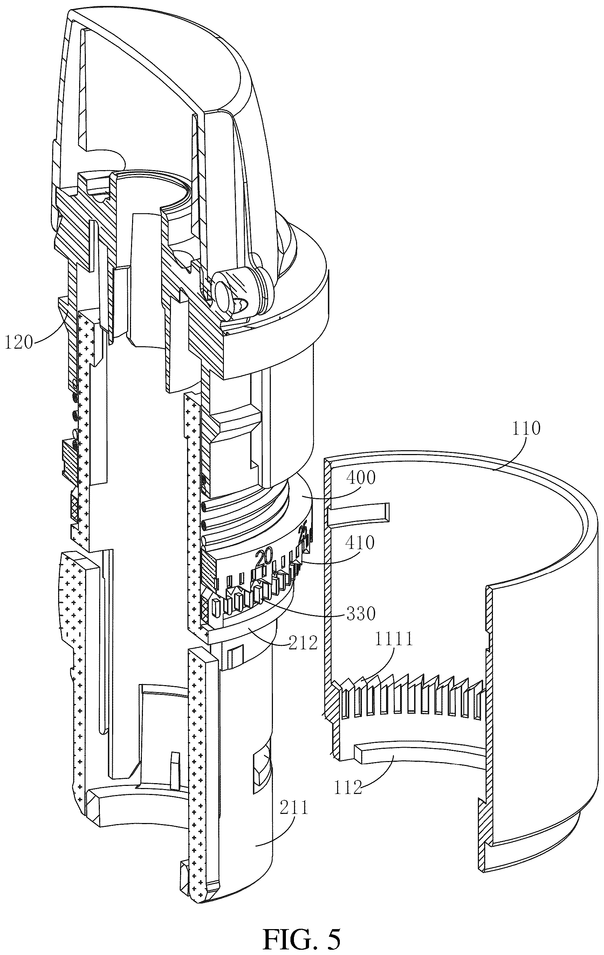

Referring to FIG. 3, FIG. 4, and FIG. 5, in some embodiments, the housing assembly 100 is spaced apart from the liquid storage bottle 20. In some instances, the housing assembly 100 and the liquid storage bottle 20 do not form a direct contact and connection relationship. In some embodiments, housing assembly 100 comprises a fixed shell 110 and a top cover 120. The fixed shell 110 is substantially of a cylindrical structure with a long axial length. For convenience of description, a direction perpendicular to the axial direction of the entire atomizer 10 is generally understood as the radial direction of the atomizer 10. The fixed shell 110 is spaced apart from the liquid storage bottle 20 at a distance in the radial direction of the atomizer 10.

The top cover 120 is arranged at the end portion of the fixed shell 110. The top cover 120 is spaced apart from the liquid storage bottle 20 at a distance in the axial direction of the atomizer 10.

The fixed shell 110 includes a fixed shell protrusion 111 and a bearing ring 112. Both the fixed shell protrusion 111 and the bearing ring 112 are arranged on the inner wall surface of the fixed shell 110 in a protruding manner, so that the fixed shell protrusion 111 and the bearing ring 112 protrude out at a length in the radial direction of the atomizer 10 relative to the inner wall surface. The fixed shell protrusion 111 and the bearing ring 112 are spaced apart from each other in the axial direction of the atomizer 10. A plurality of fixed shell protrusions 111 are included, which are arranged in the circumferential direction of the fixed shell 110. Guide teeth 1111 are arranged at the axial top of the fixed shell protrusions 111. The guide teeth 1111 have vertical guide surfaces 1111a and inclined guide surfaces 1111b. One end of each vertical guide surface 1111a and one end of each inclined guide surface 1111b are connected to each other. The axial direction of the fixed shell 110 is the same as the axial direction of the atomizer 10. The vertical guide surfaces 1111a can extend in the axial direction of the atomizer 10, and the inclined guide surfaces 1111b can tilt in an angle relative to the axial direction of the atomizer 10, so that both the vertical guide surfaces 1111a and the inclined guide surfaces 1111b are arranged in an acute angle. For two adjacent guide teeth 1111, an end portion of the inclined guide surface 1111b of one guide tooth 1111 is in contact with the vertical guide surface 1111a of the other guide tooth 1111, so that the two adjacent guide teeth 1111 form a mutual contact relationship. A gap between two adjacent guide teeth 1111 will form a guide slot 1112. Specifically, the inclined guide surface 1111b of one guide tooth 1111 and the vertical guide surface 1111a of the other guide tooth 1111 are jointly enclosed to form the guide slot 1112. Namely, the inclined guide surface 1111b of one of two adjacent guide teeth 1111 and the vertical guide surface 1111a of the other guide tooth 1111 are enclosed to form the guide slot 1112. In other embodiments, the inclined guide surface 1111b and the vertical guide surface 1111a of two adjacent guide teeth 1111 can not be in contact, so that the two adjacent guide teeth 1111 are spaced apart from each other without being in contact.

A sliding notch 1113 is provided in the inner wall surface of each fixed shell protrusion 111. The sliding notch 1113 extends in the axial direction of the atomizer 10. The sliding notch 1113 is recessed into a portion of the inclined guide surface 1111b, so that the sliding notch 1113 has an opening 1114 in the inclined guide surface 1111b.

The bearing ring 112 extends in a ring shape in the circumferential direction of an inner surface of the fixed shell 110. The bearing ring 112 is located close to the end portion of the fixed shell 110 located at the bottom. For example, the bearing ring 112 can be continuously distributed in the circumferential direction to form a closed ring, or the bearing ring 112 can be divided into a plurality of arcing segments discretely arranged in the circumferential direction. In some embodiments, bearing ring 112 comprises two or more adjacent arcing segments spaced apart by a distance in the circumferential direction.

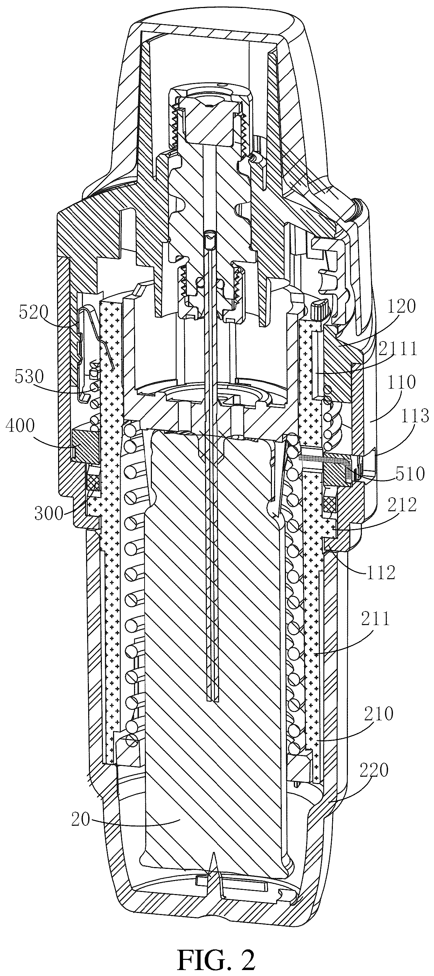

Referring to FIG. 1 and FIG. 2, a window 113 is further provided in the fixed shell 110. Window 113 is a through hole, so that window 113 penetrates through the entire fixed shell 110 in the radial direction. The fixed shell 110 further includes an indicator 114. Indicator 114 is arranged on the edge of window 113. For example, indicator 114 protrudes out of the hole wall surface of window 113, and indicator 114 protrudes out at a length in the axial direction of atomizer 10 relative to the hole wall surface.

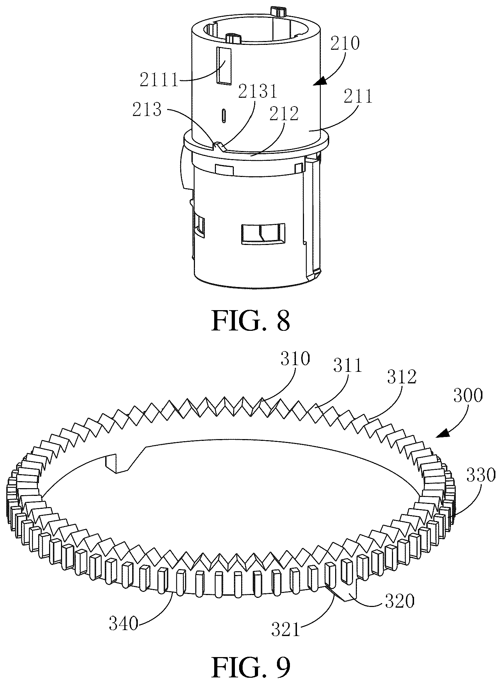

Referring to FIG. 2, FIG. 3, and FIG. 8, in some embodiments, the driving assembly 200 includes a rotating shaft 210 and a sleeve 220. The sleeve 220 fixedly covers the rotating shaft 210, so that the sleeve 220 cannot slide and rotate relative to the rotating shaft 210, thus keeping both the rotating shaft 210 and the sleeve 220 in synchronous motion. The sleeve 220 and the fixed shell 110 are arranged in the axial direction of the atomizer 10. The sleeve 220 can be in direct contact with a user, and the user can directly apply a torque to the sleeve 220, so that the sleeve 220 drives the rotating shaft 210 to rotate synchronously.

The rotating shaft 210 includes a shaft portion 211, a flange 212, and a pushing portion 213. The entire rotating shaft 210 can be integrally formed, and the axial direction of the rotating shaft 210 is the same as the axial direction of the entire atomizer 10. The shaft portion 211 is substantially of a tubular structure. The interior of the shaft portion 211 can comprise a receiving space into which liquid storage bottle 20 can be inserted, positioned, and/or housed. The shaft portion 211 is spaced apart from the liquid storage bottle 20 at a set distance in the radial direction of atomizer 10, so that the shaft portion 211 and the liquid storage bottle 20 form a non-contact relationship.

Flange 212 is positioned on an outer surface of shaft portion 211 and is circular-ringshaped in some embodiments. Flange 212 sleeves the shaft portion 211 and protrudes out at a length in the radial direction of the atomizer 10 relative to the outer surface of shaft portion 211. During assembly, the fixed shell 110 is arranged around the rotating shaft 210. The flange 212 is carried on the bearing ring 112 of the fixed shell 110. The bearing ring 112 can play an axial limiting role in the mounting of the flange 212 and the entire rotating shaft 210. The rotating shaft 210 can rotate around its own center axis relative to the fixed shell 110. The pushing portion 213 is arranged on the flange 212. The pushing portion 213 protrudes out at a length in the axial direction of the atomizer 10 relative to the surface of the flange 212. In some instances, pushing portion 213 protrudes out at a length in the axial direction of atomizer 10 from an axial-facing surface of flange 212. The pushing portion 213 has a first inclined surface 2131. The first inclined surface 2131 tilts in an angle relative to the axial direction of the atomizer 10, so that the first inclined surface 2131 is arranged in an acute angle with the axial direction of the atomizer 10. Two pushing portions 213 can be present in some embodiments. The two pushing portions 213 are spaced apart at 180° in the circumferential direction of the shaft portion 211. In some embodiments, three or more pushing portions 213 can be provided. When there are three or more pushing portions 213, all the pushing portions 213 are arranged in the circumferential direction of the shaft portion 211 in a uniform spacing manner, so that an angle between any two adjacent pushing portions 213 in the circumferential direction of the shaft portion 211 is equal.

Referring to FIG. 3, FIG. 4, and FIG. 9, in some embodiments, the jacking plate 300 can be substantially of a cylindrical structure, such as a ring-like structure, with a short axial length, and the axial direction of the jacking plate 300 is the same as the axial direction of the atomizer 10. The jacking plate 300 is nested in the fixed shell 110, so that the fixed shell 110 is arranged around the jacking plate 300. The jacking plate 300 is arranged around the shaft portion 211 of the rotating shaft 210 such that shaft portion 211 extends through the ring-like jacking plate 300. Jacking plate 300 includes a jacking tooth 310, a stop 320, and a convex strip 330. The jacking tooth 310 and the stop 320 are respectively located at the two opposite ends in the axial direction of the jacking plate 300. Specifically, stop 320 is located at an edge of the jacking plate 300 in the radial direction. In an embodiment, a plurality of the jacking teeth 310 are present. The plurality of jacking teeth 310 are arranged in the circumferential direction of the jacking plate 300. Each jacking tooth 310 can have two inclined surfaces 311. One end of one of the two inclined surfaces 311 is connected to one end of the other inclined surface 311. The two inclined surfaces 311 can tilt in an angle relative to the axial direction of the atomizer 10, so that two inclined surfaces 311 on the same jacking tooth 310 are connected to form an acute angle. For two adjacent jacking teeth 310, the end portion of the inclined surface 311 of one jacking tooth 310 is connected to the end portion of the inclined surface 311 of the other jacking tooth 310, so that the two adjacent jacking teeth 310 form a mutual contact relationship. The gap between the two adjacent jacking teeth 310 forms a jacking slot 312. Specifically, the inclined surface 311 of one jacking tooth 310 and the inclined surface 311 of the other jacking tooth 310 are jointly enclosed to form the jacking slot 312, namely, the two inclined surfaces 311 of the two adjacent jacking teeth 310 are enclosed to form the jacking slot 312. As discussed in more detail herein, jacking teeth 310 are configured to push the counting plate 400 to move.

The convex strip 330 is arranged on the outer side surface of the jacking plate 300. The convex strip 330 extends in the axial direction of the atomizer 10. The convex strip 330 protrudes out at a length in the radial direction of the atomizer 10 from an outer-facing surface of jacking plate 300. In some embodiments, a plurality of convex strips 330 are present. The plurality of convex strips 330 are spaced apart in the circumferential direction of the jacking plate 300. The number of the convex strips 330 is equal to the number of the sliding notches 1113 on the fixed shell 110, so that the convex strips 330 form a one-to-one correspondence relationship with the sliding notches 1113. The convex strips 330 are in sliding, complementary fit with the sliding notches 1113, so that the entire jacking plate 300 can slide in the axial direction of the atomizer 10 relative to the fixed shell 110.

Jacking plate 300 has an annular end surface 340. The end surface 340 is arranged around the axis of the jacking plate 300. Stop 320 is positioned on the end surface 340 of the jacking plate 300 in a protruding manner, so that the stop 320 protrudes out at a length in the axial direction of the atomizer 10 relative to the end surface 340. The stop 320 has a second inclined surface 321. The second inclined surface 321 tilts in an angle relative to the axial direction of the atomizer 10, so that the second inclined surface 321 is arranged in an acute angle with the axial direction of the atomizer 10. The second inclined surface 321 and the first inclined surface 2131 of each pushing portion 213 can have equal inclination angles relative to the axial direction of the atomizer 10. The number of stops 320 is equal to the number of the pushing portions 213, so that stops 320 and pushing portions 213 form a one-to-one correspondence relationship.

In some embodiments, two stops 320 can be included. The two stops 320 are equally spaced apart by 180° in the circumferential direction of the jacking plate.

In some embodiments, three or more stops 320 can be provided. When there are three or more stops 320, all the stops 320 are arranged in the circumferential direction of the jacking plate 300 in a uniform spacing manner, so that an angle between any two adjacent stops 320 in the circumferential direction of the jacking plate 300 is equal. After the assembling is completed, the stops 320 resist against the end surface 340 of the jacking plate 300. The stops 320 can resist against the flange 212, so that the flange 212 is spaced apart by a distance from the end surface 340 of the jacking plate 300 in the axial direction of the atomizer 10.

In an embodiment, the two inclined surfaces 311 of two adjacent jacking teeth 310 can be do not contact each other, so that the two adjacent jacking teeth 310 are spaced apart from each other without being in contact.

The jacking teeth 310 can alternatively be replaced with other rodlike structures, so long as the jacking plate 300 can push against counting plate 400 to move counting plate 400. The jacking plate 300 can also alternatively sleeve the fixed shell 110, so that the jacking plate 300 is arranged around the fixed shell 110.

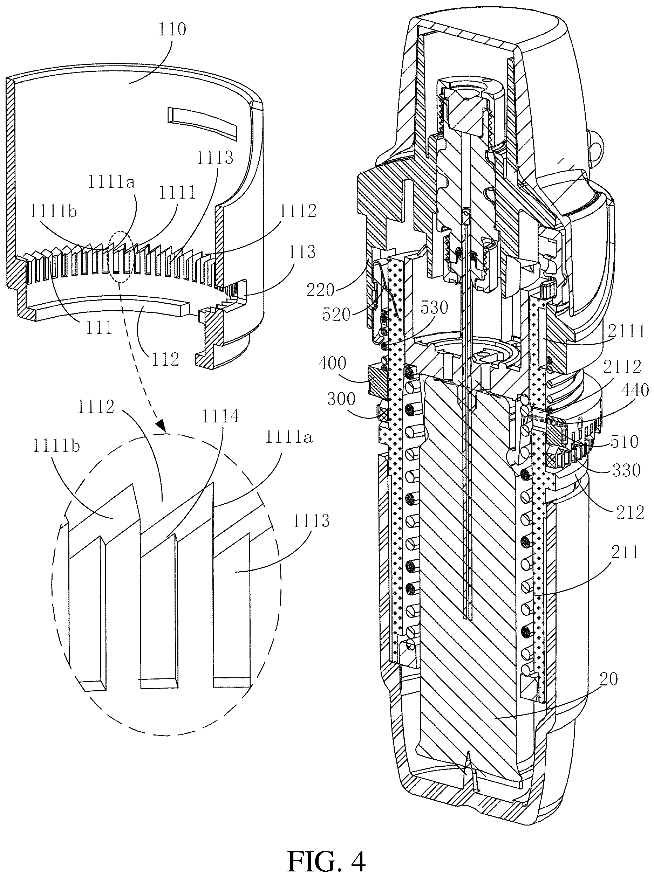

Referring to FIG. 3 and FIG. 4, during operation, sleeve 220 can be rotated clockwise to drive the rotating shaft 210 to move clockwise. When the pushing portions 213 resist against the end surface 340 of the jacking plate 300, the pushing portions 213 cannot push the jacking plate 300 to slide relative to the fixed shell 110. When the pushing portions 213 of the rotating shaft 210 are in contact with the stops 320 of the pushing plate, the first inclined surfaces 2131 and the second inclined surfaces 321 are in contact with each other. Since both the first inclined surfaces 2131 and the second inclined surfaces 321 tilt in an angle relative to the axial direction of the atomizer 10, the pushing portions 213 generate a component force in the axial direction of the atomizer 10 to the stops 320. The component force is a pushing force applied from the pushing portions 213 to the jacking plate 300, so that the pushing portions 213 push the entire jacking plate 300 to upwardly slide relative to the fixed shell 110, making the stops 320 slide upwards gradually away from the flange 212 of the rotating shaft 210. Due to the sliding fit between the convex strips 330 and the sliding notches 1113, the smoothness of the sliding of the jacking plate 300 relative to the fixed shell 110 can be improved, thereby reducing shaking and jamming generated during the upward sliding of the jacking plate 300. Additionally, in the process of the jacking plate 300 gradually moving upwards, an avoidance space is provided for the rotation of the rotating shaft 210, which effectively avoids obstruction of the stops 320 to the rotation of the rotating shaft 210.

At the moment when the pushing portions 213 pass over the stops 320 and stop the contact, the pushing portions 213 lose their supporting functions on the jacking plate 300, so that the jacking plate 300 can slide downwards under the promotion of its own gravity, and the stops 320 then gradually approach the flange 212 until the stops 320 rests against the flange 212. In some embodiments, an elastic member can be used to press against the jacking plate 300 and be quickly reset through an elastic force. Specifically, the elastic member can be a spring that coaxially sleeves an outer side of the rotating shaft 210 of the atomizer 10.

By proper setting of the number of the pushing portions 213 and the number of the stops 320, during the continuous clockwise rotation of the rotating shaft 210, the rotating shaft 210 can periodically push the jacking plate 300 to slide upwards relative to the fixed shell 110, thereby causing the jacking plate 300 to periodically move up and down relative to the fixed shell 110. For example, when there are two pushing portions 213 and two stops 320, at each rotation of 180°, the rotating shaft 210 can push the jacking plate 300 to achieve a reciprocating up-down motion relative to the fixed shell 110. When there are three pushing portions 213 and three stops 320, at each rotation of 120°, the rotating shaft 210 can push the jacking plate 300 to achieve a reciprocating up-down motion relative to the fixed shell 110.

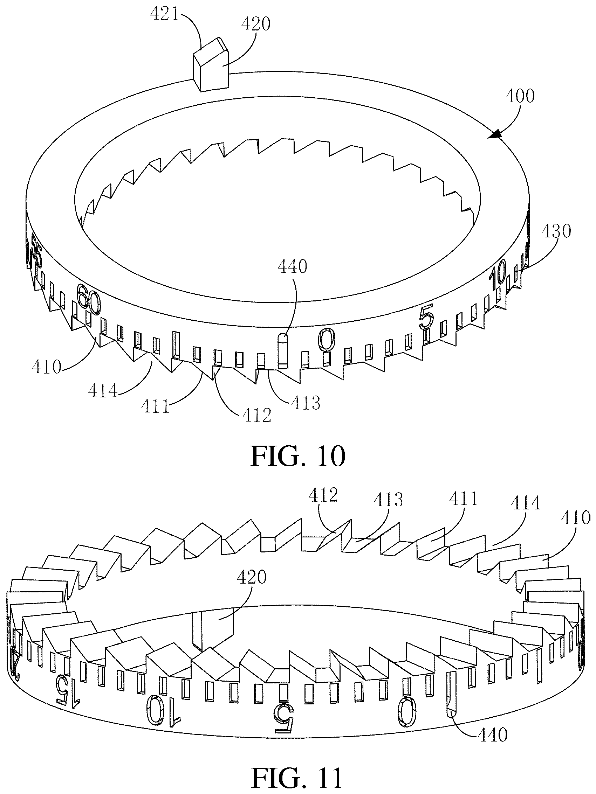

Referring to FIG. 3, FIG. 10, and FIG. 11, in some embodiments, the counting plate 400 can be substantially of a cylindrical structure with a small axial length, and the axial direction of the counting plate 400 is the same as the axial direction of the atomizer 10. The counting plate 400 is nested in the fixed shell 110, so that the fixed shell 110 is arranged around the counting plate 400, and the counting plate 400 is arranged around the shaft portion 211 of the rotating shaft 210. The counting plate 400 includes a rotating tooth 410 and a protruding portion 420. The rotating tooth 410 and the protruding portion 420 are respectively located at two opposite ends of the counting plate 400 in the axial direction. A plurality of the rotating teeth 410 are included. The plurality of rotating teeth 410 are arranged in the circumferential direction of the counting plate 400, each extending outward from an end of the counting plate 400 in the same axial direction. The rotating teeth 410 include vertical rotating surfaces 411 and inclined rotating surfaces 412. One end of each vertical rotating surface 411 and one end of each inclined rotating surface 412 are connected to each other. The vertical rotating surfaces 411 can extend in the axial direction of the atomizer 10. The inclined rotating surfaces 412 can tilt in an angle relative to the axial direction of the atomizer 10, so that a vertical rotating surface 411 and an inclined rotating surface 412 together are arranged in an acute angle.

For two adjacent rotating teeth 410, the inclined rotating surface 412 of one rotating tooth 410 and the vertical rotating surface 411 of the other rotating tooth 410 are not in contact with each other and are spaced apart by a set distance in the circumferential direction of the counting plate 400. The spatial separation of the two adjacent rotating teeth 410 forms a non-contact relationship, where the two adjacent rotating teeth 410 are spaced apart in the circumferential direction of the counting plate 400. A gap between the two adjacent rotating teeth 410 can form a receiving slot 414. Specifically, the counting plate 400 further has end surfaces 413. The end surface 413 are located between two adjacent rotating teeth 410. The rotating teeth 410 protrude out at a length in the axial direction of the atomizer 10 relative to the end surfaces 413. For two adjacent rotating teeth 410, one end of the end surface 413 is connected to the vertical rotating surface 411 of one of the rotating teeth 410, and the other end of the end surface 413 is connected to the inclined rotating surface 412 of the other rotating tooth 410, so that the end surface 413, the inclined rotating surface 412 of one of the rotating teeth 410, and the vertical rotating surface 411 of the other rotating tooth 410 are jointly enclosed to form the receiving slot 414.

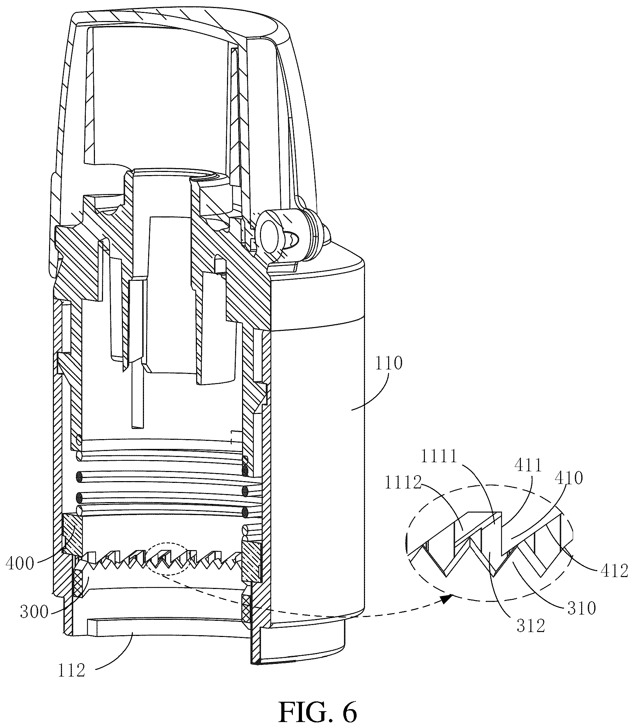

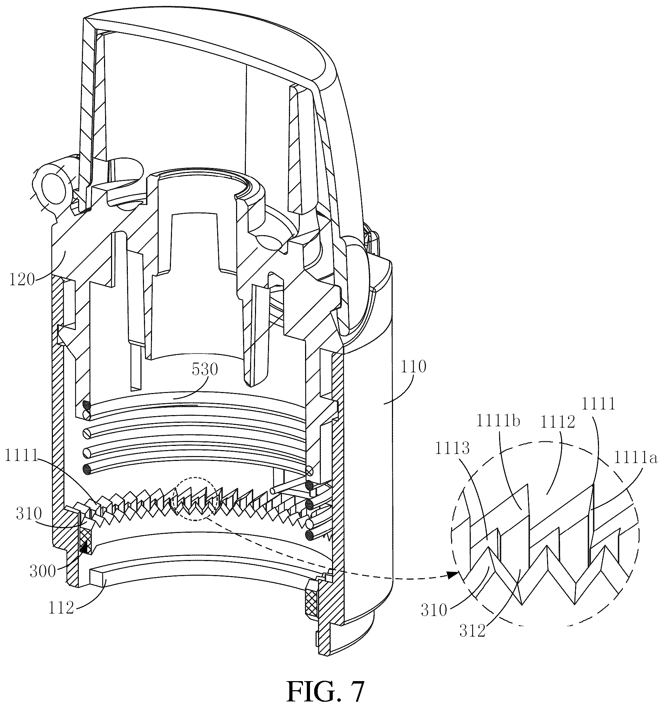

Referring to FIG. 6 and FIG. 7, the guide teeth 1111 of the fixed shell 110 match ate with the receiving slots 414, and the rotating teeth 410 match with both the jacking slots 312 and the guide slots 1112. In other embodiments, the inclined rotating surface 412 of one of two adjacent rotating teeth 410 is in contact with the vertical rotating surface 411 of the other rotating tooth 410, so that the two adjacent rotating teeth 410 are in contact with each other.

The protruding portion 420 protrudes out at a length in the axial direction of the atomizer 10 relative to a main body portion of the counting plate 400. The protruding portion 420 has a third inclined surface 421. The third inclined surface 421 tilts in an angle relative to the axial direction of the atomizer 10, and the inclination angle is less than 90°. The counting plate 400 is further provided with scale markings 430. The scale markings 430 are located on the outer side surface of the counting plate 400. For example, the scale markings 430 can be countersinks provided in the outer side surface. The countersinks extend a distance in the axial direction of the atomizer 10. A plurality of scale markings 430 can be included. The plurality of scale markings 430 are arranged in the circumferential direction of the counting plate 400 in a uniform spacing manner, so that an angle between any two adjacent scale markings 430 in the circumferential direction of the counting plate 400 is equal.

The counting plate 400 can be further provided with digits corresponding to the corresponding scale markings 430. In an embodiment, a maximum value of the above digits can be 70 and a minimum value is 0. In some instances, the scale markings 430 can alternatively be protrusions or scale lines arranged on the outer side surface of the counting plate 400.

Referring to FIG. 1, window 113 in fixed shell 110 can expose some of the scale markings 430 on the counting plate 400. A user can directly observe the scale markings 430 in window 113, and indicator 114 can point to a specific scale marking 430, so that the user can quickly and accurately determine a volume of remaining medicinal liquid in the liquid storage bottle 20, and then accurately determine a volume of ingested medicinal liquid. For example, when indicator 114 points to a scale marking 430 of "70", the medicinal liquid in the liquid storage bottle 20 has a dosage of "70" doses. When indicator 114 points to a scale marking 430 of "0", the medicinal liquid in the liquid storage bottle 20 has a dosage of "0" dose. When indicator 114 points to a scale marking 430 of "N", the medicinal liquid in the liquid storage bottle 20 has a dosage of "N" doses. In an embodiment, when indicator 114 points to the scale marking 430 of "70", it can mean that the liquid storage bottle 20 is in a saturated and full state. In this case, the medicinal liquid in the liquid storage bottle 20 has not been used in any way. When indicator 114 points to the scale marking 430 of "0", it can mean that the liquid storage bottle 20 is in a completely empty state. In this case, the medicinal liquid in the liquid storage bottle 20 has been used up.

Referring to FIG. 1, FIG. 2, and FIG. 4, in some embodiments, the counting plate 400 is provided with a first hole 440. The first hole 440 is a through-hole and penetrates through the counting plate 400 in the radial direction. For example, the first hole 440 can be provided in an extension line of the scale marking 430 with the largest digit. A second hole 2112 is provided in the shaft portion 211 of the rotating shaft 210. A diameter of the second hole 2112 can be greater than a diameter of the first hole 440. The first hole 440 can be a circular hole with a first length, and the second hole 2112 can be a circular hole with a second length that is greater than the first length. When a new liquid storage bottle 20 in the full state is placed into the atomizer 10, the first hole 440 is located within a coverage range of window 113, and the first hole 440 and the second hole 2112 are aligned with each other. In this case, it can ensure that indicator 114 just points to the scale marking 430 with the largest number, indicating that the liquid storage bottle 20 is in an initial full state. Therefore, in the process of placing the liquid storage bottle 20 in the full state into the atomizer 10, a user can adjust a positional relationship between the counting plate 400 and the rotating shaft 210. When the user can see the second hole 2112 through the first hole 440, the first hole 440 and the second hole 2112 are aligned with each other, the counting plate 400 and the rotating shaft 210 are aligned accurately, and indicator 114 just points to the scale marking 430 with the largest digit.

Further, to improve the convenience of operation, the atomizer 10 can further include a positioner 510. The positioner 510 can be simultaneously inserted into or pulled out of the first hole 440 and the second hole 2112. In the process of placing the liquid storage bottle 20 in the full state into the atomizer 10, the positional relationship between the counting plate 400 and the rotating shaft 210 is adjusted. When the positioner 510 can be simultaneously inserted into the first hole 440 and the second hole 2112, the counting plate 400 and the rotating shaft 210 are aligned accurately, which can ensure that indicator 114 just points to the scale marking 430 with the largest digit.

This can effectively avoid relying on visual inspection only whether the first hole 440 and the second hole 2112 are aligned. The positioner 510 is used to replace visual inspection to determine whether the first hole 440 and the second hole 2112 are aligned, which ultimately improves the alignment efficiency of the counting plate 400 and the rotating shaft 210. After the new liquid storage bottle 20 in the full state is placed into the atomizer 10, when the atomizer 10 is used, to prevent the positioner 510 from interfering with the rotation of the rotating shaft 210, the user can directly pull out, with a hand, the positioner 510 that is inserted into the first hole 440 and the second hole 2112, or can remove the positioner 510 out of the first hole 440 and the second hole 2112 through a strong magnet.

Referring to FIG. 3 and FIG. 4, in some embodiments, the atomizer 10 further includes an elastic clamp strip 520. The elastic clamp strip 520 can be pressed between the housing assembly 100 and the shaft portion 211 of the rotating shaft 210. One end of the elastic clamp strip 520 is slidably connected to the top cover 120 of the housing assembly 100, and the other end of the elastic clamp strip 520 is a free end and resists against the surface of the shaft portion 211. A groove 2111 is provided in the shaft portion 211. The groove 2111 can extend a length in the axial direction of the atomizer 10. In an initial state, the free end of the elastic clamp strip 520 is spaced apart from the position of the groove 2111 in the axial direction of the atomizer 10 by a distance, which can be generally understood that the groove 2111 is located above the free end of the elastic clamp strip 520. During the rotation of the rotating shaft 210, when there is still medicinal liquid in the liquid storage bottle 20, the elastic clamp strip 520 cannot match with the groove 2111 to circumferentially position the shaft portion 211, so that the free end of the elastic clamp strip 520 can not hinder the rotation of the shaft portion 211. Considering that the counting plate 400 includes the protruding portion 420, the protruding portion 420 has a third inclined surface 421. During the rotation of the rotating shaft 210, when the medicinal liquid in the liquid storage bottle 20 is used up, the protruding portion 420 can rotate to a position corresponding to the elastic clamp strip 520. The third inclined surface 421 of the protruding portion 420 can resist against the elastic clamp strip 520, and the third inclined surface 421 can exert an upward pushing force on the elastic clamp strip 520, causing the elastic clamp strip 520 to slide a distance upwards relative to the housing assembly 100. The groove 2111 of the shaft portion 211 just rotates to a position corresponding to the free end of the elastic clamp strip 520. In this case, the elastic clamp strip 520 releases energy, and finally, the free end of the elastic clamp strip 520 is clamped into the groove 2111. The elastic clamp strip 520 achieves a circumferential positioning effect on the shaft portion 211, thereby preventing the rotating shaft 210 from continuously rotating. Therefore, during the use of the atomizer 10, if the rotating shaft 210 cannot rotate, it means that the medicinal liquid in the liquid storage bottle 20 is used up.

In some embodiments, the atomizer 10 further includes an elastic member 530. The elastic member 530 sleeves the shaft portion 211 of the rotating shaft 210, and the two ends of the elastic member 530 respectively resist against the top cover 120 and the counting plate 400.

An elastic force generated by the elastic member 530 and the gravities of the jacking plate 300 and the counting plate 400 can cause the jacking plate 300 and the counting plate 400 to move downwards.

The following describes the working principle of the atomizer 10:
Referring to FIG. 3, FIG. 6, and FIG. 7, a user rotates the rotating shaft 210 through the sleeve 220. When the pushing portions 213 resist against the end surface of the jacking plate 300, the pushing portions 213 cannot push the jacking plate 300 to slide relative to the fixed shell 110, and the jacking plate 300 is in a stationary state. In this case, the rotating teeth 410 are simultaneously accommodated in the jacking slots 312 and the guide slots 1112, and the guide teeth 1111 are accommodated in the receiving slots 414. The vertical rotating surfaces 411 of the rotating teeth 410 abut against the vertical guide surfaces 1111a of the guide teeth 1111; the inclined rotating surfaces 412 of the rotating teeth 410 abut against the inclined guide surfaces 1111b of the guide teeth 1111; and the jacking teeth 310 resist against the inclined rotating surfaces 412 of the rotating teeth 410. When the first inclined surfaces 2131 of the pushing portions 213 resist against the second inclined surfaces 321 of the stops 320, the pushing portions 213 can push the jacking plate 300 to move upwards relative to the fixed shell 110, thereby causing the jacking teeth 310 to push the rotating teeth 410 and the entire counting plate 400 to move upwards by rotating the inclined surfaces. During the upward movement of the rotating teeth 410, the inclined rotating surfaces 412 of the rotating teeth 410 gradually move away from the inclined guide surfaces 1111b of the guide teeth 1111. The rotating teeth 410 move upwards in the extension direction of the vertical guide surfaces 1111a, and the counting plate 400 that moves upwards can achieve a squeezing effect on the elastic member 530, so that the elastic member 530 stores energy.

When the end portions of the rotating teeth 410 move upwards to the end portions of the guide teeth 1111, the first inclined surfaces 2131 are about to leave the second inclined surfaces 321. In this case, under the action of the elastic force of the elastic member 530, due to the guide effect of the inclined guide surfaces 1111b on the inclined rotating surfaces 412, each rotating tooth 410 is separated from one guide slot 1112 and enters another adjacent guide slot 1112. In the process that each rotating tooth 410 enter from one guide slot 1112 to another adjacent guide slot 1112, the first inclined surfaces 2131 are separated from the second inclined surfaces 321. Under the action of the gravity and the elastic force of the elastic member 530, the jacking plate 300 and the counting plate 400 both slide downwards. The counting plate 400 can rotate while sliding downwards. During the upward sliding of the counting plate 400, the atomizer 10 can not spray medicinal liquid. During the rotation of the counting plate 400, the rotation of the counting plate 400 can trigger the atomizer 10 to spray a dose of medicinal liquid in the liquid storage bottle 20. When the counting plate 400 stops rotating, the counting plate 400 stops triggering the atomizer 10 to spray the medicinal liquid, and indicator 114 points to a new scale marking 430 (see FIG. 1). Therefore, the user can accurately know the volume of the liquid sprayed by the atomizer 10 and the volume of the remaining liquid in the liquid storage bottle 20 through the scale marking 430 pointed to by indicator 114 every time the counting plate 400 rotates.

For example, before the counting plate 400 rotates, indicator 114 points to a scale marking 430. This scale marking 430 is referred to as a first scale marking. Using a first scale marking of "55" as an example, liquid of 55 doses remains in the liquid storage bottle 20. When the rotating shaft 210 is rotated to make the counting plate 400 rotate once, indicator 114 points to a next scale marking 430. This scale marking 430 is referred to as a second scale marking. In this example, the second scale marking would correspond to a digit of "54", indicating one less liquid dose (than the original 55 doses) remains in the liquid storage bottle 20. Therefore, during the rotation of the counting plate 400, the atomizer 10 sprays the medicinal liquid of one dose, namely, the user ingests the medicinal liquid of one dose. Therefore, through the continuous rotation of the rotating shaft 210 and the scale markings 430 pointed to by indicator 114, the dosage of the medicinal liquid sprayed by the atomizer 10 during the rotation time of the rotating shaft 210 and the dosage of the medicinal liquid remaining in the liquid storage bottle 20 can be accurately known.

Each time the rotating shaft 210 rotates at a set angle, the jacking plate 300 can be pushed to move and slide upwards once, so that the counting plate 400 can rotate once, and then the atomizer 10 can spray a dose of the medicinal liquid. For example, in a case that two pushing portions 213 are provided, every time the rotating shaft 210 rotates 180°, the jacking plate 300 can be pushed to move and slide upwards once, and the counting plate 400 can rotate once. The angle of one rotation of the counting plate 400 can be an angle between two or more adjacent scales in the circumferential direction. When the angle of one rotation of the counting plate 400 is the angle between two adjacent scales in the circumferential direction, the medicinal liquid sprayed by the atomizer 10 during each rotation of the counting plate 400 is in a dosage of one dose. When the angle of one rotation of the counting plate 400 is the angle between three or four adjacent scales in the circumferential direction, the medicinal liquid sprayed by the atomizer 10 during each rotation of the counting plate 400 is in a dosage of two doses or three doses.

When the atomizer 10 sprays all the medicinal liquid in the liquid storage bottle 20, indicator 114 can point to the scale marking 430 corresponding to "0". Meanwhile, the protruding portion 420 can rotate to the position corresponding to the elastic clamp strip 520. The protruding portion 420 can generate an upward-movement pushing force on the elastic clamp strip 520, so that the elastic clamp strip 520 slides upwards a distance relative to the housing assembly 100. Then, the free end of the elastic clamp strip 520 can be inserted into the groove 2111 of the shaft portion 211 so that the entire rotating shaft 210 plays a locking role, and the user will be unable to continuously rotate the rotating shaft 210.

In some embodiments, the number of the guide teeth 1111 is a quotient obtained by dividing the maximum digit corresponding to the scale marking 430 by an odd number. The number of the jacking teeth 310 can also be a quotient obtained by dividing the maximum digit corresponding to the scale marking 430 by an odd number. Through this setting, the number of the guide teeth 1111 and the number of the jacking teeth 310 can be appropriately reduced, so as to lower the processing difficulty of a mold, reduce the manufacturing costs of the atomizer 10, and improve the smoothness of movement of the counting plate 400. Exemplarily, if the maximum designed digit is "69", the number of the guide teeth 1122 is "69" according to a maximum ratio. The odd value mentioned above is "1". Further, to lower the processing difficulty of the mold and reduce the manufacturing costs of the atomizer 10, the odd value mentioned above is "3", which means that the maximum designed digit is "69", and the number of the guide teeth 1122 is "23". In this example, the number of the guide teeth 1122 has decreased, but the same effect of "69" pieces of digits has been achieved. Similarly, when the maximum designed digit is another value, other corresponding value relationships can be established. For example, when the maximum digit is "60", the number of the guide teeth 1122 can be equal to "60", "20", or "12". Certainly, the number of the guide teeth 1122 should not be too small as a small number can affect the stability and smoothness of rotation. Therefore, preferably, the odd number is one of 1, 3, and 5.

The various technical features in the foregoing embodiments can be randomly combined. For concise description, not all possible combinations of the various technical features in the above embodiments are described. However, provided that combinations of these technical features do not conflict with each other, the combinations of the various technical features are considered as falling within the scope of this specification.

The foregoing embodiments merely express several implementations of this application. The descriptions thereof are relatively specific and detailed, but should not be understood as limitations to the scope of this application. A person of ordinary skill in the art can also make several transformations and improvements without departing from the idea of this application. These transformations and improvements fall within the protection scope of this application. Therefore, the protection scope of the patent of this application shall be subject to the appended claims.

## Claims

1. An atomizer, comprising:
a housing assembly comprising a fixed shell comprising
an indicator,
a plurality of guide teeth arranged in a circumferential direction, and
a guide slot is formed between two adjacent guide teeth;
a jacking plate slidably connected to the fixed shell in an axial direction of the housing assembly;
a counting plate comprising
a plurality of rotating teeth arranged in the circumferential direction,
a receiving slot formed between two adjacent rotating teeth, and
scale markings arranged in the circumferential direction;
wherein the rotating teeth match with the guide slots; the guide teeth match with the receiving slots; and
a driving assembly movably connected to the fixed shell and contacting a radial edge of the jacking plate, wherein the jacking plate slides when a force is exerted thereon by the driving assembly, causing the counting plate to rotate and the indicator to point to different scale markings.

2. The atomizer of claim 1, wherein both the counting plate and the jacking plate are nested in the fixed shell.

3. The atomizer of claim 1, further comprising an elastic member, wherein the housing assembly further comprises a top cover; the top cover is connected to the fixed shell and is spaced apart from the counting plate in the axial direction; and the elastic member is pressed between the top cover and the counting plate.

4. The atomizer according to claim 3, wherein the elastic member is a spring, and the spring sleeves the driving assembly.

5. The atomizer of claim 1, wherein the driving assembly comprises a rotating shaft comprising:
a shaft portion rotatably nested in the jacking plate and the counting plate and surrounding a liquid storage bottle,
a flange sleeving the shaft portion, and
a pushing portion positioned on the flange in a protruding manner in the axial direction of the shaft portion;
wherein when the shaft portion rotates, the pushing portion pushes the jacking plate to slide.

6. The atomizer of claim 5, wherein
the pushing portion has a first inclined surface arranged in an acute angle with an axial direction of the shaft portion;
the jacking plate comprises a stop located at an end portion thereof, the stop having a second inclined surface arranged in an acute angle with the axial direction of the shaft portion;
wherein the first inclined surface of the pushing portion and the second inclined surface of the jacking plate periodically press against each other.

7. The atomizer of claim 6, further comprising:
a plurality of the stops and a plurality of the pushing portions, both being uniformly spaced in a circumferential direction,
wherein the number of the stops and the number of the pushing portions are equal.

8. The atomizer of claim 5, wherein the driving assembly further comprises a sleeve fixed over the shaft portion; the sleeve and the fixed shell being positioned in the axial direction.

9. The atomizer of claim 1, further comprising
an elastic clamp strip pressed between the housing assembly and the driving assembly and connected on a first end to the housing assembly; and
a groove formed in the driving assembly;
wherein when a second end of the elastic clamp strip opposite the first end is inserted into the groove, the driving assembly is prevented from moving.

10. The atomizer of claim 9, wherein:
the elastic clamp strip is slidably connected to the housing assembly;
the counting plate comprises a protruding portion arranged at an end away from the rotating teeth, the protruding portion having a third inclined surface; and
wherein when the counting plate rotates, the third inclined surface slides the elastic clamp strip into the grove until the second end of the elastic clamp strip is inserted into the groove.

11. The atomizer of claim 1, wherein the jacking plate comprises
a plurality of jacking teeth arranged in the circumferential direction; and
a plurality of jacking slots, each jacking slot being formed between two adjacent jacking teeth;
wherein the jacking slots match with the rotating teeth.

12. The atomizer of claim 11, wherein each jacking tooth comprises two inclined surfaces that are connected to each other and arranged at an angle with the axial direction of the jacking plate, the two inclined surfaces on the same jacking tooth form an acute angle, and the two inclined surfaces of two adjacent jacking teeth are connected to each other and are enclosed to form the jacking slot.

13. The atomizer of claim 1, wherein:
the fixed shell further comprises a window exposing the scale markings,
the indicator is positioned on an edge of the window,
a first hole is disposed in the counting plate, and
a second hole is disposed in the driving assembly;
wherein when a new liquid storage bottle is mounted into the atomizer, the first hole is located within the window and is aligned with the second hole.

14. The atomizer of claim 13, further comprising a positioner that is simultaneously insertable into or removable out of the first hole and the second hole when the new liquid storage bottle is mounted into the atomizer.

15. The atomizer of claim 14, wherein a diameter of the second hole is greater than a diameter of the first hole; the first hole is circular with a first length; and
the second hole is circular with a second length that is greater than the first length.

16. The atomizer of claim 1, wherein the fixed shell comprises a fixed shell protrusion arranged on the inner wall surface of the fixed shell, the fixed shell protrusion comprising the guide teeth, the guide teeth having:
vertical guide surfaces, and
inclined guide surfaces connected to the vertical guide surfaces;
wherein the vertical guide surfaces are parallel to the axial direction of the fixed shell, the inclined guide surfaces form an acute angle with the vertical guide surfaces, and the vertical guide surface of one of two adjacent guide teeth and the inclined guide surface of the other guide tooth are enclosed to form the guide slot.

17. The atomizer of claim 16, wherein the fixed shell protrusion comprises a sliding notch that is recessed into the inclined guide surfaces, the jacking plate comprises a convex strip protruding out of the outer side surface of the jacking plate, and the convex strip is complementary in shape with the sliding notch.

18. The atomizer of claim 16, wherein for two adjacent guide teeth, an end portion of the inclined guide surface of one guide tooth is in contact with the vertical guide surface of the other guide tooth.

19. The atomizer of claim 1, wherein the rotating teeth comprise vertical rotating surfaces and inclined rotating surfaces connected to the vertical rotating surfaces, the vertical rotating surfaces extending in the axial direction of the counting plate, the inclined rotating surfaces forming an acute angle with the vertical rotating surfaces, and for two adjacent rotating teeth, the vertical rotating surface of one rotating tooth and the inclined rotating surface of the other rotating tooth are spaced apart from each other at a set distance in the circumferential direction of the counting plate and are configured to be enclosed to form the receiving slot.

20. The atomizer according to claim 1, wherein the number of the guide teeth is a quotient obtained by dividing a maximum digit corresponding to the scale markings by an odd number.
